(19)

Europäisches Patentamt
European Patent Office
Office européen des brevets

(11) **EP 3 586 803 A1**

(12) **EUROPEAN PATENT APPLICATION**

(43) Date of publication:
01.01.2020 Bulletin 2020/01

(51) Int Cl.:
*A61F 7/08* (2006.01)   *A47G 9/02* (2006.01)
*B32B 15/20* (2006.01)   *B32B 3/10* (2006.01)

(21) Application number: 18179987.5

(22) Date of filing: 26.06.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: **The Surgical Company International B.V.**
3821 AH  Amersfoort (NL)

(72) Inventor: **VAN OUDENALLEN, Robertus Gerardus**
3452 CM Vleuten (NL)

(74) Representative: **Hindles Limited**
**Clarence House**
**131-135 George Street**
**Edinburgh, EH2 4JS (GB)**

(54) **TEMPERATURE-REGULATION APPARATUS**

(57)    The invention provides a temperature-regulation apparatus with a segmented metal-containing layer. The metal-containing layer has an electrically insulating coating and a plurality of mutually electrically insulated planar metal segments to increase insulation by reducing thermal emissivity whilst minimizing the hazards of an electrically conductive layer. The temperature-regulation apparatus may comprise a blanket which may be used to actively supply temperature regulated air for regulating the temperature of a subject, in which case it includes at least one port, or it may be used to passively maintain the temperature of a subject, or it may comprise a temperature-regulation garment.

Fig.1

EP 3 586 803 A1

## Description

Field of the invention

The invention relates to the field of temperature-regulation apparatuses.

Background to the invention

Temperature-regulation apparatuses are used in both hypo- and hyperthermia treatments to help to regulate or maintain the temperature of a subject. These devices typically take the form of temperature regulation blankets, including passive drapes with a metal layer (such as "space blankets"), forced air warming blankets comprising a gas impermeable sheet with a metal layer and a gas permeable sheet through which temperature regulated air is forced in use, or they take the form of temperature regulation garments. In each case, the purpose of the metal layer is to increase insulation by reflecting heat back to the subject.

As a metal layer is electrically conductive there are associated skin burn hazards, as well as fire hazards, if such an apparatus is used with electrical equipment. For this reason, conventional temperature-regulation apparatuses typically have an electrically insulating coating surrounding the metal layer.

Furthermore, if such temperature-regulation apparatuses are used over therapy electrodes, such as those used in defibrillation equipment, the metal layer can become capacitively charged, which can then lead to an uncontrolled discharge.

Accordingly, the invention seeks to avoid these disadvantages of known temperature-regulation apparatus having sheets with a metal layer.

Summary of the invention

A first aspect of the invention provides a temperature-regulation apparatus for the temperature regulation of a (e.g. human) subject (typically in contact therewith), the apparatus comprising a sheet, the sheet comprising a metal-containing layer between insulating coating layers, the metal-containing layer comprising one or more metal-containing regions, the or each metal-containing region comprising a plurality of mutually electrically insulated planar metal segments.

Because the metal-containing layer comprises planar metal segments which are mutually electrically insulated (i.e. electrically insulated from each other), the sheet does not become electrically conductive, across the plane of the sheet, in the event that the insulating coating fails.

Furthermore, the individual segments can be made small enough that the capacitive charge imparted to each of the metal segments in use (for example during a defibrillation procedure) is too small to overcome the permeability of the insulating coating, thus preventing any uncontrolled discharge.

The or each metal-containing region may comprise more than 100 mutually electrically insulated planar metal segments. Thus, the metal in the metal-containing layer is typically divided into at least 100 parts.

Preferably, the (mutually electrically insulated planar metal) segments each have, on average (mean) an area of less than 1000 mm$^2$, typically less than 500 mm$^2$, or less than 200 mm$^2$. Preferably, the (mutually electrically insulated planar metal) segments each have, on average (mean) an aspect ratio of maximum to minimum dimension in the plane of the segment of less than 10: 1, or less than 5:1. Preferably, the (mutually electrically insulated planar metal) segments each have, on average (mean) a thickness of less than 0.1 $\mu$m, typically less than 0.05 $\mu$m thick or less than 0.025 $\mu$m. The (mutually electrically insulated planar metal) segments (within a said metal containing region) are typically separated by at least 0.1 mm or at least 0.2 mm. The average (mean) spacing between the metal segments within the or each metal-containing region is typically less than 2 mm or less than 1 mm or less than 0.5 mm (i.e. to thereby avoid large non-metal spaces between metal segments). Where there is a plurality of metal containing regions, the metal containing regions are distinct from each other, typically being separated by a gap greater than the spaces between metal segments.

The metal-containing layer is thermally insulating because it reflects heat. Therefore it is preferable if the mutually electrically insulated planar metal segments of the metal-containing layer are arranged such that there is a relatively high proportion of the total area of the temperature-regulation apparatus that comprises the mutually electrically insulated planar metal segments and, correspondingly, a relatively low proportion that comprises the spaces between the mutually electrically insulated planar metal segments. For example, it may be that the (metal) segments occupy 90% or more of the surface area of the metal-containing layer. It may be that the (metal) segments occupy 90% or more of the or each metal-containing region. It may be that the ratio of the surface area of the metal segments to the surface area of the spaces between the metal segments is at least 10:1. It may be that the (metal) segments occupy 90% or more of the surface area of the sheet.

As such, it is preferable if the shapes of the mutually electrically insulated planar metal segments are chosen such that they tessellate, (i.e. to thereby avoid wasting space). Therefore, in some the metal segments comprise tessellating shapes, the shapes being equally spaced. Example shapes for the mutually electrically insulated planar metal segments therefore include, but are not limited to, triangles, squares and hexagons. Similarly, it is preferable if the mutually electrically insulated planar metal segments are equally spaced.

In some embodiments, the (mutually electrically insulated planar metal) segments of one metal-containing region differ in size and/or spacing and/or shape from at least one or each other metal-containing region. For example, one metal-containing region may comprise mutually electrically insulated planar metal segments which are larger and/or have a smaller spacing therebetween than another said metal-containing region. However, it may be that the mutually electrically insulated planar metal segments in different metal-containing regions correspond in size, spacing and/or shape.

In some embodiments the temperature-regulation apparatus may comprise (e.g. be) a forced air warming product. In some embodiments the temperature-regulation apparatus may comprise (e.g. be) a temperature-regulation blanket (e.g. a forced air warming blanket).

In some embodiments the temperature-regulation apparatus may comprise (e.g. be) an underbody, that is to say, it may be configured such that a portion of (or in some embodiments all of) the apparatus is suitable for use beneath a subject (typically a human subject, optionally a human patient), for example it may comprise a recess to receive a subject's head.

In some embodiments the temperature-regulation apparatus further comprises an additional sheet (which may be non-metallized), the additional sheet being sealedly attached to the said sheet (comprising a metal-containing layer), thereby defining a chamber therebetween. The additional sheet is typically air-permeable (e.g. porous). In such embodiments the temperature-regulation apparatus typically further comprises an inlet port, e.g. for the supply of temperature-regulated air into said chamber, thus allowing active regulation of the temperature of a subject. The inlet port is typically formed in the sheet comprising the metal-containing layer or in the additional sheet. Thus, the temperature regulation apparatus may be a forced-air (warming) product.

In some embodiments the temperature-regulation apparatus may comprise (e.g. be) a temperature-regulation garment (typically a forced-air warming garment). In some embodiments the temperature regulation garment may comprise sleeves and/or fastening means. In some embodiments the garment may comprise (e.g. be) a hospital gown. In some embodiments the temperature regulation garment may comprise a removable access panel.

Typically, the insulating coating(s) on one or both sides of the metal-containing layer (optionally on one or both sides of the planar metal segments) comprises (e.g. multiple) layers of polypropylene (PP), polyethylene (PE) and polyurethane (PU) and/or adhesives (to thereby secure the insulating coating to the metal-containing layer). Typically, the metal segments are formed as a layer of aluminium which is either deposited or sheet transferred onto a substrate, such as PP, PE or PU.

The insulating coating(s) on both sides of the metal-containing layer (optionally on one or both sides of the planar metal segments) may be in contact with each other in the spaces between the metal segments to thereby mutually electrically insulate the metal segments. However, electrically insulating material may be provided between the insulating coatings, in the spaces between the metal segments. Typically, the metal segments (and/or the metal containing layer) comprise (e.g. are) aluminium.

When used, any inlet ports in the temperature-regulation apparatus typically have port connectors which can be coupled to a hose, pipe or any other suitable transfer means for the supply of air, which may be temperature-regulated air.

The sleeves, access panel and/or fastening means where present, may be porous. The sleeves, access panel and/or fastening means may comprise non-woven (e.g. PP and PE) film or other suitable materials. The sleeves, access panel and/or fastening means may be detachable from the temperature-regulation apparatus.

In some embodiments wherein the apparatus comprises an additional sheet, the additional sheet may be detachable from (and typically re-attachable to) the temperature-regulation apparatus (for example it may be attached via buttons, zips, press-studs, ties, Velcro, etc.).

Description of the Drawings

An example embodiment of the present invention will now be illustrated with reference to the following Figures in which:

Figure 1 is a plan view of an example embodiment wherein the temperature-regulation apparatus comprises a blanket;

Figure 2 is a plan view of an example embodiment wherein the temperature-regulation apparatus comprises a blanket, with a plurality of distinct metal-containing regions;

Figure 3 is a cross-section through a basic temperature-regulation apparatus;

Figure 4 is a cross-section through a temperature-regulation apparatus further comprising an additional sheet sealedly attached to the temperature-regulation apparatus, thereby defining a chamber, as inflated;

Figure 5 is a more detailed example cross-section through a temperature-regulation apparatus;

Figure 6 is a front view of an example embodiment wherein the temperature-regulation apparatus comprises a garment;

Figure 7 is a back view of an example embodiment wherein the temperature-regulation apparatus comprises a garment.

Detailed Description of an Example Embodiment

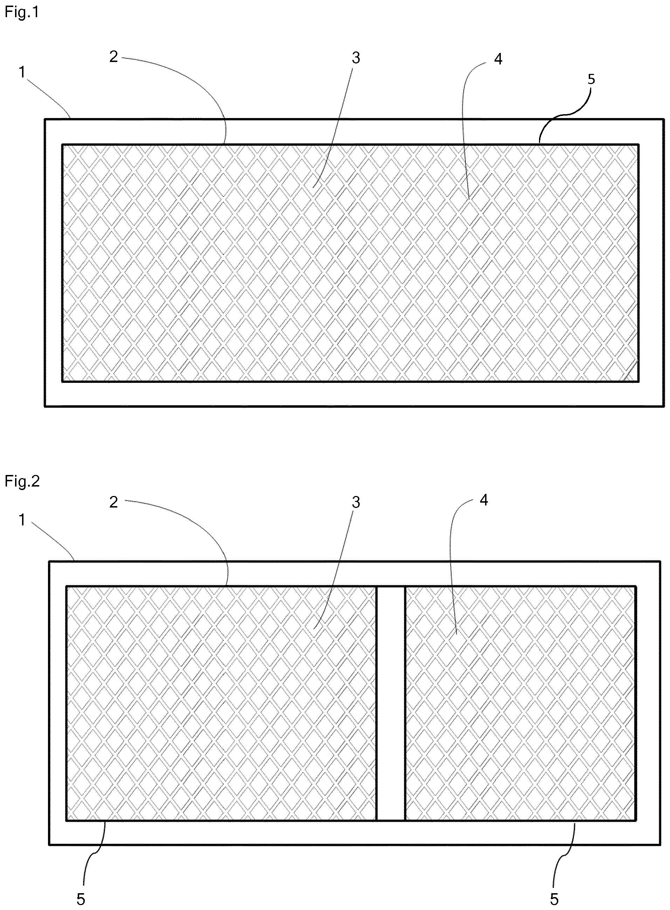

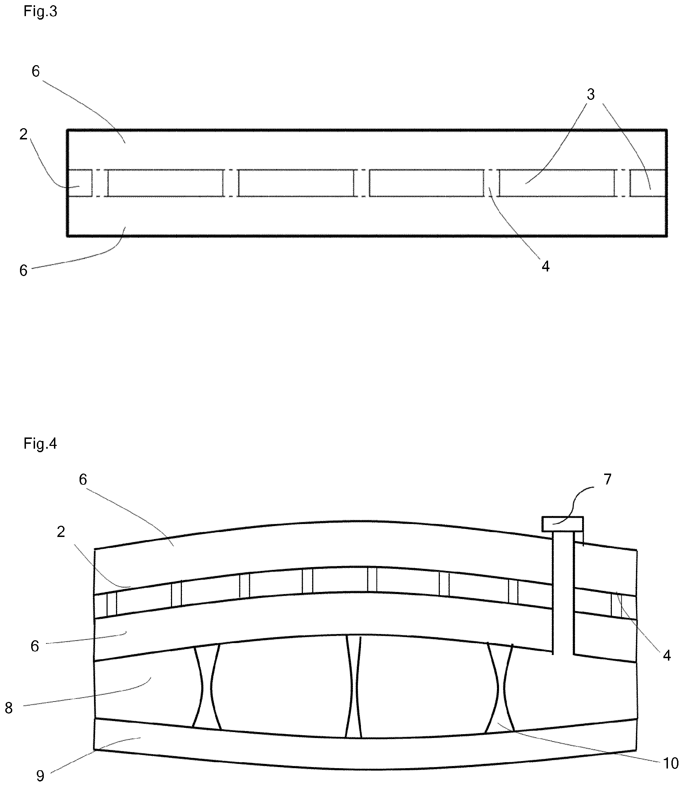

With reference to Figure 1, a temperature-regulation apparatus according to the invention is a blanket in the form of a sheet (1). The sheet (1) has a metal-containing layer (2) with one or more distinct metal-containing regions (5) (Figure 2 is a diagram of an example of a temperature-regulation blanket with multiple distinct regions (5)). Each region (5) has at least 100 planar metal segments (3), with spaces (4) in between adjacent segments (3). Electrically insulating coating layers (6) surround the metal containing layer (2) and these coating layers (6) are in contact with each other in the spaces (4) in between adjacent segments (3), as can be seen in Figure 3, which is a cross-section through a basic temperature-regulation blanket.

Each metal segment (3) covers, on average, an area of less 1000 mm, is less than 0.05 μm thick, and is separated from adjacent segments (3) by at least 0.2 mm. Combined, the metal segments (3) cover at least 90% of the total surface area of the blanket. The segments (3) tessellate (they are rhomboid in shape) and are evenly spaced apart.

The metal-containing layer (2) of the blanket is thermally insulating in use because it reflects heat back towards a subject. In this way the temperature of the subject can be passively maintained. The spaces (4) between the segments (3) ensure that each segment (3) is electrically insulated from the segments (3) adjacent to it. The segments (3) themselves are each small enough that the capacitive charge imparted to a segment (3) during a defibrillation procedure is too small to overcome the permeability of the coatings (6), thus preventing any uncontrolled discharge.

Suitable dimensions for the planar metal segments are found according to the following equation, in which $A_{segment}$ is the area of an individual segment (3), $A_{impact}$ is the area of a potential burn due to an uncontrolled discharge, $\rho$ is the energy density of such an uncontrolled discharge, $d$ is the thickness of the insulating coatings (6), $\varepsilon$ is the permittivity of free space, $\kappa$ is the relative permittivity and $V$ is the voltage applied during a defibrillation procedure.

$$A_{segment} = \frac{2\, A_{impact}\, \rho\, d}{\varepsilon\, \kappa\, V^2}$$

Further to this, suitable dimensions for the spaces (4) between adjacent planar metal segments (3) may be found according to the following equation, in which $\delta$ is the spacing between adjacent segments (3), $V$ is the voltage applied during a defibrillation procedure and $h$ is the dielectric strength of the insulating coatings (6).

$$\delta = \frac{V}{h}$$

For example, in a typical defibrillation procedure a voltage, $V$, of 5000 V is used. In an example case where the insulating coatings (6) have a dielectric strength, $h$, this would imply the choice of a distance, $\delta$, of at least 5000/h, between adjacent segments (3). For example, if such a temperature regulation blanket were intended for use during a 5000 V defibrillation procedure, and the dielectric strength of the coatings was 20 MV/m, the distance between adjacent metal segments would be at least 0.25 mm.

The metal in the metal-containing layer (2) is aluminium and is sheet transferred onto a plastic surface such as a polypropylene (PP) (11), polyethylene (PE) (13) or polyurethane (PU) (17) substrate. The insulating coatings (6) are made up of multiple layers of PP (11), PE (13) and PU (17) and are joined to each other, and to the metal-containing layer (2), with adhesives (16). One skilled in the art will appreciate that other electrically insulating materials may be used in place of PP (11), PE (13) and/or PU (17).

Because of the coatings (6) the blanket cannot conduct electricity through its plane and because of the spaces (4) between the segments (3) the blanket cannot conduct electricity across its plane. As a result, the blanket is safer than other known blankets with a metal containing region that do not feature either insulating coatings or mutually insulated metal segments, since the risks of fire, burning and/or electrocution are reduced when the blanket is used around electrical appliances or defibrillation equipment.

With reference to Figure 4, which shows a cross-section through a temperature-regulation blanket of the general type shown in Figure 3, the blanket also has an additional sheet (9). This additional sheet (9) is attached to the first sheet (1), to form a chamber (8). The two sheets are held together via fastening means (10) and the first sheet (1) has an inlet port (7). In this configuration, the temperature-regulation blanket can be used as a forced-air temperature-regulation blanket. The fastening means may be welds between the first sheet and the additional sheet.

The chamber (8) can be filled with temperature-regulated air via the inlet port (7) (which has a port connector for the attachment of a hose, for example, for supplying temperature-regulated air). The temperature-regulated air can slowly leave the chamber (8) via the additional sheet (9), which is porous and thus air-permeable.

This configuration of the temperature-regulation blanket allows active control of the temperature of a subject (8) by regulating the temperature of the air which is supplied into the chamber and which is forced through the porous additional sheet towards the subject. This is advantageous in the treatment of hypo- or hyperthermia. The additional sheet (9) may in some embodiments be detachable from the temperature-regulation blanket, should active control of the subject's temperature not be necessary.

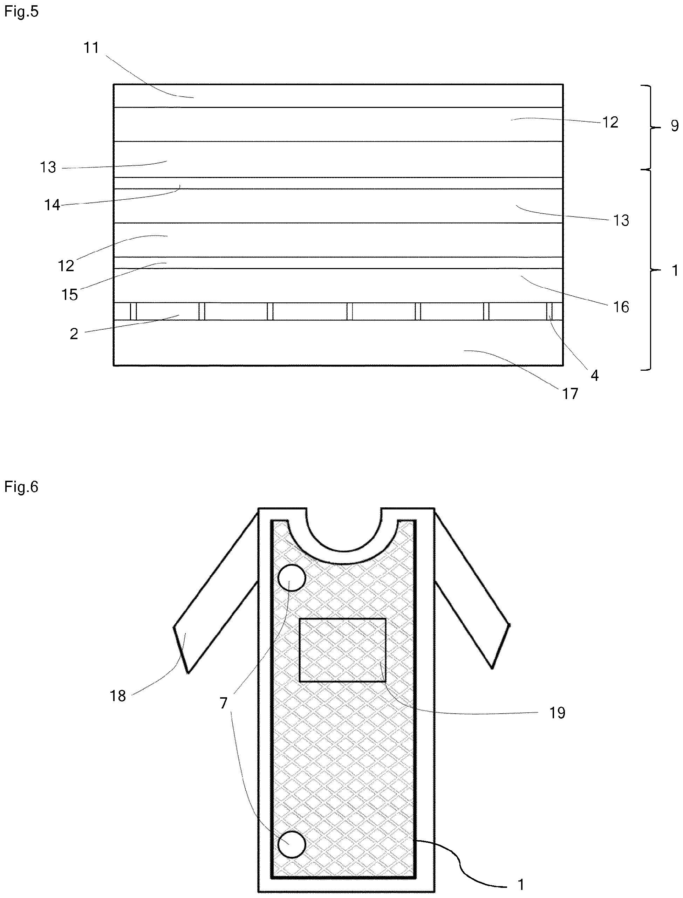

Figure 5 is a more detailed example cross-section through a temperature-regulation blanket of the general type shown in Figure 3. Here it can be seen that the insulating coating (6) has layers of PU (17), PP (11) and PE (13), as well as adhesive (16), a sealing treatment (12) and a binding treatment (18). It can also be seen that the additional sheet (9) has layers of PE (13) and PP (11) as well as a binding treatment (14). These layers combine to make an electrically insulating coating layer (6) that has a dielectric strength great enough to prevent the flow of electricity through or across the blanket, as well as preventing uncontrolled discharge.

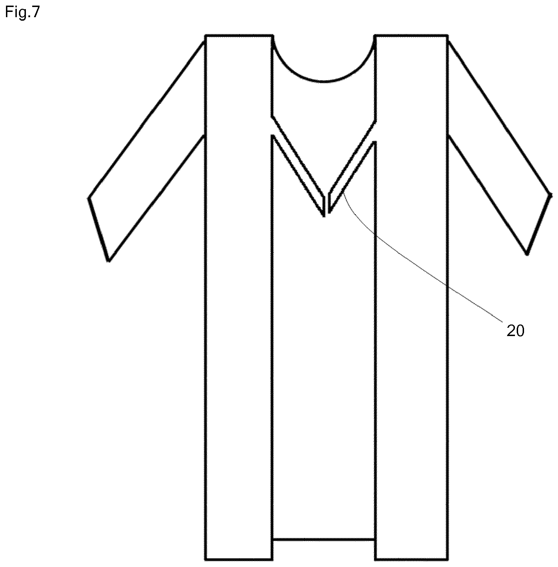

In a further example embodiment, the temperature-regulation apparatus (1) comprises a garment, as shown in Figure 6. In such an example, the garment takes the form of a hospital gown for a subject to wear. The gown has sleeves (18), an access panel for use during surgery (19) and fastening means (20), as shown in Figure 7, such as ties or buttons. The temperature of a subject wearing the gown could be passively controlled while the subject moved around, or, if the subject was stationary and the inlet ports (7) were in use for the supply of temperature regulated air, the temperature of the subject could be actively controlled.

**Claims**

1. A temperature-regulation apparatus comprising a sheet, the sheet comprising a metal-containing layer between electrically insulating coating layers, the metal-containing layer comprising one or more metal-containing regions, the or each metal-containing region comprising a plurality of mutually electrically insulated planar metal segments.

2. A temperature-regulation apparatus according to claim 1 wherein the or each metal-containing region comprises more than 100 mutually electrically insulated planar metal segments.

3. A temperature-regulation apparatus according to any one preceding claim wherein the segments each have, on average (mean) an area of less than 1000 mm$^2$ and/or wherein the segments each have on, average (mean) a thickness of less than 1 $\mu$m.

4. A temperature-regulation apparatus according to any one preceding claim wherein the segments are separated by at least 0.1 mm.

5. A temperature-regulation apparatus according to any one preceding claim wherein the segments occupy 90% or more of the surface area of the sheet.

6. A temperature-regulation apparatus according to any one preceding claim wherein the metal segments comprise tessellating shapes, said shapes being equally spaced.

7. A temperature-regulation apparatus according to any one preceding claim wherein the segments of one metal-containing region differ in size and/or spacing and/or shape from at least one or each other metal-containing region.

8. A temperature-regulation apparatus according to any one preceding claim wherein the apparatus is a forced air warming product.

9. A temperature-regulation apparatus according to any one preceding claim wherein the apparatus is a temperature-regulation blanket.

10. A temperature-regulation apparatus according to claim 9 wherein the temperature regulation apparatus comprises an underbody, configured such that a portion of the apparatus is suitable for use beneath a subject.

11. A temperature-regulation apparatus according to any one preceding claim wherein the temperature regulation apparatus further comprises an additional sheet, the additional sheet being sealedly attached to the said sheet, thereby defining a chamber therebetween, the temperature regulation apparatus further comprising an inlet port, wherein the additional sheet is air-permeable.

12. A temperature-regulation apparatus according any one preceding claim wherein the insulating coating(s) on one or both sides of the metal containing comprises layers of polypropylene (PP), polyethylene (PE), polyurethane (PU), and/or adhesives, optionally wherein the insulating coating(s) on one or both sides of the metal containing layer are in contact with each other in the spaces between said segments.

13. A temperature-regulation apparatus according to any one preceding claim wherein the insulating coatings on both sides of the metal segments are in contact with each other in the spaces between said segments.

14. A temperature-regulation apparatus according to any one preceding claim wherein the metal segments are aluminium.

15. A temperature regulation apparatus according to any one preceding claim wherein the apparatus comprises a temperature-regulation garment, optionally wherein the garment comprises a hospital gown.

Fig.1

Fig.2

Fig.3

Fig.4

Fig.5

Fig.6

Fig.7

20

Europäisches
Patentamt

European
Patent Office

Office européen
des brevets

## EUROPEAN SEARCH REPORT

Application Number

EP 18 17 9987

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/298117 A1 (GILES ANDREW JAMES [US] ET AL) 29 November 2012 (2012-11-29) | 1,2,4,7, 9,10, 12-15 | INV. A61F7/08 A47G9/02 |
| Y | * paragraphs [0002], [0024] - [0026], [0041], [0042], [0045], [0049], [0051], [0052]; figures 4, 6 * | 8,11 | B32B15/20 B32B3/10 |
| X | US 2011/262699 A1 (YIALIZIS ANGELO [US] ET AL) 27 October 2011 (2011-10-27) * paragraphs [0003], [0008], [0036], [0039], [0047], [0059] - [0063]; figures 1, 9 * | 1-7,9, 12-14 | |
| Y | EP 2 893 910 A1 (SURGICAL COMPANY INTERNAT B V [NL]) 15 July 2015 (2015-07-15) * paragraphs [0052], [0053], [0058] - [0060]; figures 1, 4a * | 8,11 | |

TECHNICAL FIELDS
SEARCHED      (IPC)

A61F
B32B
A47G

The present search report has been drawn up for all claims

| Place of search | Date of completion of the search | Examiner |
|---|---|---|
| The Hague | 12 December 2018 | Rosander, Frida |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 17 9987

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2018

| Patent document cited in search report | | Publication date | Patent family member(s) | | Publication date |
|---|---|---|---|---|---|
| US 2012298117 | A1 | 29-11-2012 | US 2012298117 A1<br>WO 2012161870 A1 | | 29-11-2012<br>29-11-2012 |
| US 2011262699 | A1 | 27-10-2011 | NONE | | |
| EP 2893910 | A1 | 15-07-2015 | EP 2893910 A1<br>JP 2015131113 A<br>US 2015196422 A1 | | 15-07-2015<br>23-07-2015<br>16-07-2015 |

EPO FORM P0459

For more details about this annex : see Official Journal of the European Patent Office, No. 12/82